# EUROPÄISCHE PATENTSCHRIFT

(11) **EP 0 492 629 B1**
(45) Veröffentlichungstag und Bekanntmachung des Hinweises auf die Patenterteilung: **20.09.1995**
(21) Anmeldenummer: 91122231.3
(22) Anmeldetag: 24.12.1991
(51) Int. Cl.: C07D 241/44, C07D 405/12, A01N 43/60

(54) **Verfahren zur Herstellung optisch aktiver Chinoxalinyloxyphenoxypropionsäureester**
Process for preparing optically active quinoxalinyloxyphenoxypropionic acid esters
Procédé de préparation d'esters d'acides quinoxalinyloxyphénoxypropioniques optiquement actifs

(30) Priorität: 28.12.1990 DE 4042098
(43) Veröffentlichungstag der Anmeldung: 01.07.1992
(73) Patentinhaber: Hoechst Schering AgrEvo GmbH, 13342 Berlin (DE)
(72) Erfinder: Zeiss, Hans-Joachim, Dr., W-6231 Sulzbach (DE); Mildenberger, Hilmar, Dr., W-6233 Kelkheim (DE)

(56) Entgegenhaltungen:
- EP-A- 0 042 750
- EP-A- 0 060 607
- GB-A- 2 042 539
- JP-A-61 083 144
- US-A- 4 687 849
- CHEMICAL ABSTRACTS, vol. 104, no. 13, 31. M rz 1986, Columbus, Ohio, US; abstract no. 109578m, G. SAKATA ET AL. 'Synthesis and herbicidal activity of optically active ethyl 2-Ä4-(6 -chloro-2-quinoxalinyloxy)phenoxyÜpropanoa te.' Seite 721 ;

## Beschreibung

Gegenstand der vorliegenden Erfindung ist ein Verfahren zur Herstellung optisch aktiver D-Chinoxalinyloxyphenoxypropionsaeureester der allgemeinen Formel I 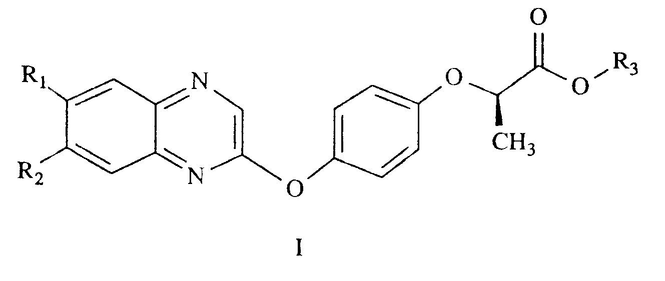
worin
- R₁: H, Halogen, vorzugsweise Chlor, C₁-C₄-Haloalkyl, vorzugsweise CF₃, CN, NO₂, C₁-C₄-Alkyl, vorzugsweise CH₃, oder C₁-C₄-Alkoxy, vorzugsweise OCH₃, insbesondere Halogen,
- R₂: H oder Halogen, vorzugsweise H,
- R₃: C₁-C₁₈-Alkyl, Benzyl, C₅-C₈-Cycloalkyl, C₃-C₄-Alkenyl, das unsubstituiert oder durch ein oder mehrere Halogenatome substituiert ist, C₃-C₄-Alkinyl, das unsubstituiert oder durch ein oder mehrere Halogenatome substituiert ist, oder (CH₂)ₙ(O)ₘR₄,
- R₄: C₁-C₆-Alkyl, das unsubstituiert oder durch C₁-C₆-Alkoxy substituiert ist, oder einen 5- oder 6-gliedrigen Ring, der gesaettigt oder ungesaettigt ist, carbocyclisch ist oder heterocyclisch mit 1 bis 4 Heteroatomen aus der Gruppe Sauerstoff und Stickstoff ist und unsubstituiert oder durch 1-3 Substituenten aus der Gruppe Oxo, C₁-C₃-Alkyl, C₁-C₃-Alkoxy substituiert ist, oder eine Gruppierung der allgemeinen Formel N=CR₅R₆,
- R₅ und R₆: unabhaengig voneinander
C₁-C₆-Alkyl, C₃-C₈-Cycloalkyl, C₁-C₆-Alkylcarbonyloxy-C₁-C₆-alkyl, C₁-C₆-Alkoxy, C₁-C₆-Alkylthio oder zusammen mit dem Kohlenstoffatom, an das sie geknuepft sind, einen Cycloalkylrest mit 3-8 Ringgliedern,
- n: 1 oder 2 und
- m: 0 oder 1
bedeuten,
dadurch gekennzeichnet, dass man
ein substituiertes Chinoxalin der allgemeinen Formel II, 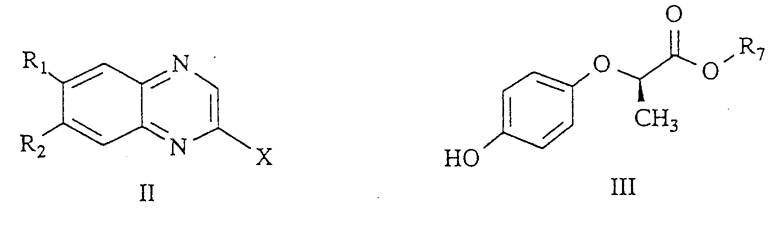
worin
- R₁ und R₂: die gleiche Bedeutung wie in Formel I haben und
- X: eine Abgangsgruppe, wie z. B. Halogen, insbesondere Chlor und Brom, sowie Methansulfonyl,
ist,
mit einem (D)-2-(4-hydroxyphenoxy)propionat der allgemeinen Formel III, worin
- R₇: C₁-C₄ Alkyl
ist, in einem unpolaren organischen Lösungsmittel in Gegenwart von 0,05 bis 1,0 mol%, bezogen auf die Verbindung der allgemeinen Formel II, eines Katalysators aus der Gruppe der Polyethylenglykole und deren Niederalkylether umsetzt, und für den Fall, daß R₃ ungleich R₇ ist, den so erhaltenen Niederalkylester der allgemeinen Formel IV bzw. der Formel I, worin R₃ = R₇ ist, 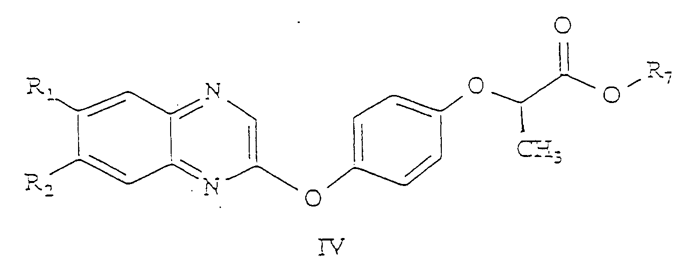
ganz oder überwiegend ohne Racemisierung zur gewünschten Verbindung der Formel I umestert. Hier und im folgenden bedeutet Halogen Fluor, Chlor, Brom oder Iod; Alkyl kann geradkettig oder verzweigt sein, entsprechendes gilt für die ungesättigten Reste Alkenyl und Alkinyl sowie die Alkylteile der substituierten Reste; niederes Alkyl ist C₁-C₆-Alkyl, vorzugsweise C₁-C₄-Alkyl.

Von besonderem Interesse ist das erfindungsgemäße Verfahren, worin in Formel I
- R₃: C₁-C₈-Alkyl, Benzyl, C₅-C₆-Cycloalkyl, C₃-C₄-Alkenyl, C₃-C₄-Alkinyl oder (CH₂)ₙ(O)ₘR₄, vorzugsweise C₁-C₄-Alkyl, CH₂CH₂ON=C(CH₃)₂ oder Tetrahydrofurfuryl,
- R₄: C₁-C₄-Alkyl, (C₁-C₆-Alkoxy)-C₁-C₄-Alkyl, einen 5- oder 6-gliedrigen gesaettigten oder ungesaettigten Ring, der carbocyclisch oder heterocyclisch mit 0 bis 3 Sauerstoffatomen und 0 bis 2 Stickstoffatomen als Ringatomen von maximal 3 Heteroringatomen ist und unsubstituiert oder durch 1-3 Substituenten aus der Gruppe Oxo, Methyl, Ethyl, n- und i-Propyl, Methoxy, Ethoxy und n- und i-Propoxy substituiert ist, oder N=CR₅R₆,
- R₅ und R₆: unabhaengig voneinander C₁-C₄-Alkyl, C₅-C₆-Cycloalkyl, C₁-C₄-Alkylcarbonyloxy-C₁-C₄-alkyl, C₁-C₄-Alkoxy, C₁-C₄-Alkylthio oder zusammen mit dem Kohlenstoffatom, an das sie geknuepft sind, einen Cycloalkylrest mit 5-6 Ringgliedern,
- n: 1 oder 2 und
- m: 0 oder 1
bedeuten.

Die im gegebenen Fall im Anschluß an die Umsetzung der Verbindungen der Formeln II und III durchzuführende Umeesterung wird vorzugsweise unter Verwendung eines Alkohols der allgemeinen Formel V

R₃OH V

worin R₃ die gleiche Bedeutung wie in Formel I besitzt, in Gegenwart eines Katalysators durchgeführt.

Verbindungen der allgemeinen Formel I werden z.B. in der DE-A-3004770 (US-A-4,629,493), der EP-A-042750 (US-A-4,609,396), der EP-A-52798 (US-A-4,435,207), der EP-A-288275 (US-A-4,948,421), der EP-A-323727 (ZA-A-88/9732) sowie der EP-A-383613 beschrieben und finden als selektiv wirksame Herbizide gegen eine grosse Anzahl wirtschaftlich interessanter Unkraeuter Verwendung.

In den meisten Faellen werden jedoch Racemate zur Anwendung gebracht, obwohl aus der EP-A-2800 bekannt ist, daß in der Klasse der substituierten Aryloxy- oder Heteroaryloxy-phenoxypropionsaueren die Enantiomeren mit D-Konfiguration eine erheblich hoehere Wirkung als die L-Enantiomeren oder die Racemate besitzen. Die dadurch moegliche Reduzierung der Aufwandmenge bei gleicher biologischer Wirkung ist sowohl aus wirtschaftlicher wie auch aus oekologischer Sicht von grossem Vorteil.

Die Synthese optisch aktiver Verbindungen der allgemeinen Formel I ist aber bisher nur fuer R₃ = H und CH₃ (DE-A-3004770 (US-A-4,629,493)), R₃ = CH₂CH₃ (J. Pesticide Sci. 1985, 10, 69), R₃ = (H₃C)₂C=N-O-(CH₂)ₙ mit n = 1 oder 2 (EP-A-52798 (US-A-4,435,207)) sowie R₃ = Tetrahydrofurfuryl (EP-A-383613) beschrieben worden.

Optisch aktive Verbindungen der Formel I werden bisher im wesentlichen nach zwei Verfahren hergestellt, und zwar, indem man entweder 2-(4-Hydroxyphenoxy)-chinoxaline VI und die entsprechenden L-Milchsaeurederivate VII (DE-A-3004770 (US-A-4,629,493); J. Pesticide Sci. 1985, 10, 69; EP-A-52798 (US-A-4,435,207)) oder aber im Falle von R₃ = Tetrahydrofurfuryl substituierte Chinoxaline der Formel II und das spezielle Tetrahydrofurfuryl-(D)-2-(4-hydroxyphenoxy)-propionat der Formel III, worin R₇ = Tetrahydrofurfuryl ist, miteinander umsetzt.

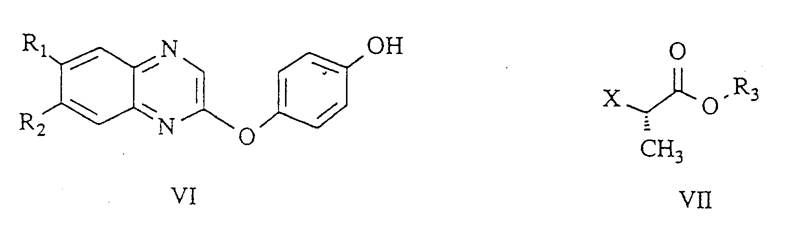

Beide Verfahren besitzen erhebliche Nachteile.

So sind zum Beispiel Verbindungen der allgemeinen Formel VI nur ueber mehrere Stufen unter Abspaltung von Schutzgruppen, wie zum Beispiel der Benzylgruppe, zugaenglich. Da bei der Umsetzung von VI mit VII teilweise Racemisierung eintritt, ist die optische Reinheit der auf diese Weise hergestellten Verbindungen der allgemeinen Formel I meist nicht hoeher als 79% (J. Pesticide Sci. 1985, 10, 69).
Die Reaktion von der Verbindungen der Formel II mit Verbindungen der Formel III, worin R₇ = Tetrahydrofurfuryl ist, fuehrt zwar zu Produkten mit hoeherer optischer Reinheit, besitzt aber den Nachteil, dass nur Verbindungen der Formel I mit R₃ = Tetrahydrofurfuryl erhalten werden koennen, also die Variationsbreite der Esterfunktion sehr gering ist. Ausserdem ist auch die Herstellung von Verbindungen der Formel III, worin R₇ = Tetrahydrofurfuryl ist, ein komplizierter, mehrstufiger Prozess (EP-OS 383613).

Verbindungen der allgemeinen Formel III mit R₇ = C₁-C₄-Alkyl sind dagegen in grossen Mengen zur Verfuegung stehende Zwischenprodukte, deren Herstellung z. B. in der JP-A-62178543 beschrieben ist.

JP-A-61083144 beschreibt die Herstellung von Ethyl (D)-2-[4-(6-chlor-2-chinoxalinyloxy) phenoxy] propionat in Acetonitril aus 2, 6-Dichlorchinoxalin und Ethyl (D)-2-(4-hydroxyphenoxy)-propionat.

Wegen der Nachteile der erwähnten bekannten Verfahren stellt das erfindungsgemäße Verfahren einen erheblichen technischen Fortschritt dar. Es gestattet, Verbindungen der allgemeinen Formel I in ihrer ganzen Variationsbreite ausgehend von Verbindungen der allgemeinen Formel III ueber eine einfach zugaengliche Zwischenstufe in hoher optischer Reinheit herzustellen.

Die Umsetzung der Verbindungen der allgemeinen Formel II mit den Verbindungen der allgemeinen Formel III erfolgt vorzugsweise in einem unpolaren, organischen Loesungsmittel in Gegenwart eines Saeurebinders, gegebenenfalls unter Zugabe eines Katalysators.

Unter unpolaren, organischen Loesungsmitteln werden bei Raumtemperatur fluessige Kohlenwasserstoffe, die auch halogenhaltig sein koennen, verstanden, wie z. B. Heptan, Toluol, Xylol, Cumol, Mesitylen, Chlorbenzol oder die isomeren Dichlorbenzole.

Als saeurebindende Mittel werden allgemein organische und anorganische Basen bevorzugt jedoch anorganische Basen verwendet. Besonders bevorzugt sind hierbei Alkalicarbonate oder Alkalihydrogencarbonate, insbesondere die des Natriums und die des Kaliums. Alkalihydrogencarbonate spalten oberhalb von ca. 90 °C Wasser ab. Um Nebenreaktionen durch Hydrolyse zu vermeiden, ist es daher bei Verwendung von Alkalihydrogencarbonaten oberhalb dieser Reaktionstemperaturen vorteilhaft, das Reaktionswasser durch geeignete Massnahmen, wie z. B. azeotrope Destillation oder Zugabe eines physikalisch wirkenden Trockenmittels wie z. B. Molekularsieb zu entfernen. Die gleichen Massnahmen empfehlen sich bei der Verwendung von Alkalicarbonaten oberhalb von ca. 90 °C, da aus diesen durch Neutralisation waehrend der Reaktion Alkalihydrogencarbonate entstehen.

Um in den oben genannten unpolaren Loesungsmitteln einen vollstaendigen Umsatz zu erzielen, kann es unter Umstaenden sinnvoll sein, einen Katalysator zuzusetzen.

Als Katalysatoren kommen Polyalkylenglykole sowie deren Niederalkylether in Frage. Katalysatorsysteme dieser Art sind im einzelnen in der EP-A-105494 beschrieben.

Vorzugsweise werden wegen der besseren Phasentrennung bei der waessrigen Aufarbeitung langkettige Polyalkylenglykole mit Molekulargewichten zwischen 1000 und 3000 eingesetzt.

Die Katalysatoren werden vorzugsweise in Mengen von 0.05 bis 1.0 mol%, insbesondere in Mengen von 0.1 bis 0.7 mol%, bezogen auf die Verbindung der allgemeinen Formel II verwendet.

Das erfindungsgemaesse Verfahren wird vorzugsweise im Temperaturbereich von 30 °C bis 150 °C, insbesondere im Bereich von 80 °C bis 120 °C, durchgefuehrt. Die Reihenfolge der Zugabe der Reaktionspartner ist in der Regel nicht kritisch.
Das Verfahren wird beispielsweise so durchgefuehrt, dass man die Reaktionspartner der allgemeinen Formeln II und III in dem gewuenschten Loesungsmittel miteinander vermischt, gegebenenfalls den Katalysator zusetzt und die gesamte Reaktionsmischung auf die gewuenschte Reaktionstemperatur erhitzt. Der Zeitpunkt, an dem ein vollstaendiger Umsatz erreicht ist, laesst sich mit gaengigen Methoden, z. B. durch duennschichtchromatographische (DC) oder gaschromatographische (GC) Untersuchung der Reaktionsmischung feststellen.

Um einen vollstaendigen Umsatz der Komponente der allgemeinen Formel II zu erzielen, ist es ratsam, einen geringen Ueberschuss der Komponente mit der allgemeinen Formel III zu verwenden. Der Ueberschuss an Komponente III bezogen auf Komponente II betraegt vorzugsweise 1 bis 10 mol%, insbesondere 1,5 bis 5 mol% und kann nach beendeter Reaktion leicht durch eine Waesche mit verduennter Alkalilauge oder einer Phosphatpufferloesung entfernt werden.

Nach Abdestillieren des Loesungsmittels werden die End- bzw. Zwischenprodukte der allgemeinen Formel I mit R₃ = R₇ bzw. Formel IV in der Regel in Ausbeuten von ueber 90 % der Theorie und optischen Reinheiten von 90 % erhalten. Sie können gewünschtenfalls isoliert oder ohne weitere Reinigung direkt zu weiteren Verbindungen der Formel I umgeestert werden. Die möglichst racemisierungsfreie Umesterung wird vorzugsweise wie folgt durchgefuehrt:

Das Zwischenprodukt der allgemeinen Formel IV wird beispielsweise in einem 2 bis 100-fachen Ueberschuss des Alkohols der allgemeinen Formel V, vorzugsweise in einem 2 bis 50-fachen Ueberschuss, geloest und nach Zugabe eines Katalysators auf 80 °C bis 120 °C erhitzt. Gegebenenfalls
kann die Reaktionsmischung durch Zugabe eines inerten Loesungsmittels verduennt werden.

Als Katalysatoren können dabei Titan-IV-tetraalkoxide eingesetzt werden, wie sie in Synthesis 1982, 138 beschrieben sind. Insbesondere geeignet sind dafür Titan-IV-ethylat, Titan-IV-isopropylat und Titan-IV-butylat. Die Menge an eingesetztem Katalysator betraegt beispielsweise 2 bis 50 mol% bezogen auf das Zwischenprodukt der allgemeinen Formel IV.

Der Zeitpunkt, an dem ein vollstaendiger Umsatz erreicht ist, laesst sich mit gängigen Methoden, z. B. DC oder GC, feststellen. Vor der Aufarbeitung kann die im Ueberschuss eingesetzte Alkoholkomponente der allgemeinen Formel V z. B. durch Destillation zurueckgewonnen werden.

Durch Aufnehmen der Reaktionsmischung nach der Umesterung in verduennter Salzsaeure werden die Titan-IV-alkoxide zerstoert und das Produkt der allgemeinen Formel I kann anschliessend durch Extraktion in ein organisches Loesungsmittel, z. B. aus der Gruppe der bei der Umsetzung der Verbindungen der Formeln II und III einsetzbaren Loesungsmittel, isoliert. Das auf diese Weise erhaltene Rohprodukt kann, falls dies gewuenscht wird, durch Massnahmen wie Umkristallisation oder Chromatographie weiter gereinigt werden.

Zur Vermeidung von Nebenreaktionen durch Luftsauerstoff oder Feuchtigkeit wird das erfindungsgemäße Verfahren vorzugsweise unter einer Schutzgasatmosphaere durchgefuehrt. Als Schutzgase kommen Stickstoff oder Argon in Betracht.

Die optischen Ausbeuten der nach dem erfindungsgemäßen Verfahren, inklusive Umesterung, erhaltenen Verbindungen der allgemeinen Formel I sind im allgemeinen über 85%, entsprechend etwa 85% enantiomeric excess.

### Beispiel 1

### Ethyl-(D)-2-[4-(6-chlor-2-chinoxalinyloxy)phenoxy]propionat

5,00 g (25 mmo]) 2,6-Dichlorchinoxalin, 5,40 g (25,8 mmol) Ethyl-(D)-2-(4-hydroxyphenoxy)-propionat (optische Reinheit: 90,2%) und 3,50 g (25 mmol) Kaliumcarbonat werden in 15 ml Xylol suspendiert und nach Zugabe von 0,3 g (0,15 mmol) Polyethylenglykol (mittl. Molekulargeweicht 2000) 6 Stunden am Wasserabscheider unter Stickstoff auf 95 °C erhitzt. Nach dieser Zeit ist im Duennschichtchromatogramm kein 2.6-Dichlorchinoxalin mehr nachweisbar. Der Ansatz wird auf Raumtemperatur abgekuehlt, filtriert und das Filtrat im Vakuum eingeengt. Man erhaelt 8,49 g (91,1% der Theorie) Ethyl-(D)-2-[4-(6-chlor-2-chinoxalinyloxy)-phenoxy]propionat als beigen Feststoff.
[α]_{D}^{20,5}=29,43 ° (c = 1,15, CHCl₃)
Bezogen auf [α]_{D}²⁰ = 35,9 ° (c = 1,20, CHCl3) fuer optisch reines Material ( J. Pesticide Sci. 1985, 10, 75) entspricht dies einer optischen Reinheit von 82 %.
Beruecksichtigt man die optische Reinheit des als Ausgangsmaterial benutzten Ethyl-(D)-2-(4-hydroxyphenoxy)-propionats (90,2%), so wuerde man ausgehend von 100 %-igem Ethyl-(D)-2-(4-hydroxyphenoxy)-propionat Ethyl-(D)2[4(6chlor-2-chinoxalinyloxy) phenoxy]-propionat in einer optischen Reinheit von 90,9 % erhalten.

### Beispiel 2

### (±)-Tetrahydrofurfuryl-(D)-2-[4-(6-chlor-2-chinoxalinyloxy)-phenoxy]-propionat

3,73 g (10 mmol) Ethyl-(D)-2-[4-(6-chlor-2-chinoxalinyloxy)-phenoxy]-propionat (aus Beispiel 1) und 1,14 g (4 mmol) Titan-IV-isopropylat werden in 50 g (48,9 mmol) (±)-Tetrahydrofurfurylalkohol geloest und 11 Stunden auf 105 °C erhitzt. Nach dieser Zeit kann duennschichtchromatographisch kein Ausgangsmaterial mehr nachgewiesen werden. Nach Abkuehlen auf Raumtemperatur wird die Reaktionsmischung mit 20 ml 1 N HCl versetzt und das Produkt durch Ausschuetteln mit 100 ml Toluol extrahiert. Die Toluolphase wird einmal mit 20 ml gesaettigter Natriumhydrogencarbonatloesung und einmal mit 20 ml Wasser gewaschen. Nach Trocknen ueber Magnesiumsulfat und Abziehen des Loesungsmittels im Vakuum werden 3,8 g (88,6% der Theorie) (±)-Tetrahydrofurfuryl-(D)-2-[4-(6-chlor-2-chinoxalinyloxy)-phenoxy]-propionat als hellbraunes Oel erhalten.
[α]_{D}²⁰ = 27,3 ° (c = 2,34, CHCl₃)
¹H-NMR (CDCL₃) δ 1,41-2,12 (m, 4, CHC**H**₂C**H**₂CH₂O), 1,65 (d, 3, J= 7 Hz, OCHC**H**₃COO), 3,61-4,01 (m, 2, OC**H**₂CHO), 4,01-4,40 (m, 3, OCH₂C**H**OC**H**₂CH₂CH₂), 4,82(q, 1, J = 7 Hz, OC**H**CH₃COO), 6,84-7,25 (m, 4, arom.), 7,48-7,77 (m, 2, arom.), 8,02 (d, 1, J= 2,5 Hz, arom.), 8,65 (s, 1, arom.).

## Patentansprüche

1. Verfahren zur Herstellung optisch aktiver D-Chinoxalinyloxy-phenoxypropionsaeureester der Formel I 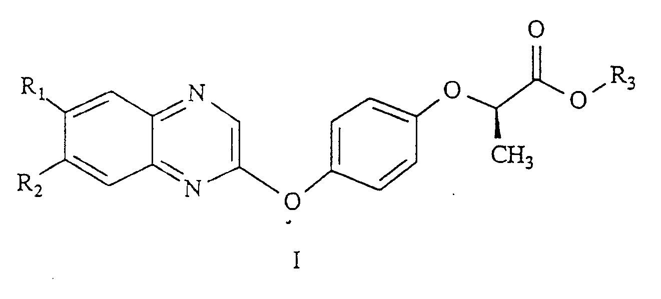 worin
R₁ H, Halogen, C₁-C₄-Haloalkyl, CN, NO₂, C₁-C₄-Alkyl, oder C₁-C₄-Alkoxy,
R₂ H oder Halogen,
R₃ C₁-C₁₈-Alkyl, Benzyl C₅-C₅-Cycloalkyl, C₃-C₄-Alkenyl, das unsubstituiert oder durch ein oder mehrere Halogenatome substituiert ist, C₃-C₄-Alkinyl, das unsubstituiert oder durch ein oder mehrere Halogenatome substituiert ist, oder (CH₂)ₙ(O)ₘR₄,
R₄ C₁-C₆-Alkyl, das unsubstituiert oder durch C₁-C₆-Alkoxy substituiert ist, oder einen 5- oder 6-gliedrigen Ring, der gesaettigt oder ungesaettigt ist, carbocyclisch ist oder heterocyclisch mit 1 bis 4 Heteroatomen aus der Gruppe Sauerstoff und Stickstoff ist und unsubstituiert oder durch 1-3 Substituenten aus der Gruppe Oxo. C₁-C₃-Alkyl, C₁-C₃-Alkoxy substituiert ist, oder eine Gruppe der allgemeinen Formel N=CR₅R₆,
R₅ und R₆ unabhaengig voneinander C₁-C₆-Alkyl, C₃-C₈-Cycloalkyl, C₁-C₆-Alkylcarbonyloxy-C₁-C₆-alkyl, C₁-C₆-Alkoxy, C₁-C₆-Alkylthio oder zusammen mit dem Kohlenstoffatom, an das sie geknuepft sind, einen Cycloalkylrest mit 3-8 Ringgliedern,
n 1 oder 2 und
m 0 oder 1
bedeuten,
dadurch gekennzeichnet, dass man
ein substituiertes Chinoxalin der allgemeinen Formel II, 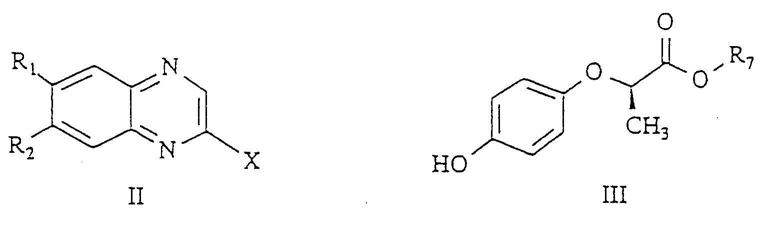 worin
R₁ und R₂ die gleiche Bedeutung wie in Formel I haben und
X eine Abgangsgruppe, wie z. B. Halogen, insbesondere Chlor und Brom, sowie Methansulfonyl,
ist,
mit einem (D)-2-(4-hydroxyphenoxy)propionat der allgemeinen Formel III, worin
R₇ C₁-C₄ Alkyl
ist, in einem unpolaren organischen Lösungsmittel in Gegenwart von 0,05 bis 1,0 mol%, bezogen auf die Verbindung der allgemeinen Formel II, eines Katalysators aus der Gruppe der Polyethylenglykole und deren Niederalkylether umsetzt, und für den Fall, daß R₃ ungleich R₇ ist, den so erhaltenen Niederalkylester der allgemeinen Formel IV bzw. der Formel I, worin R₃ = R₇ ist, 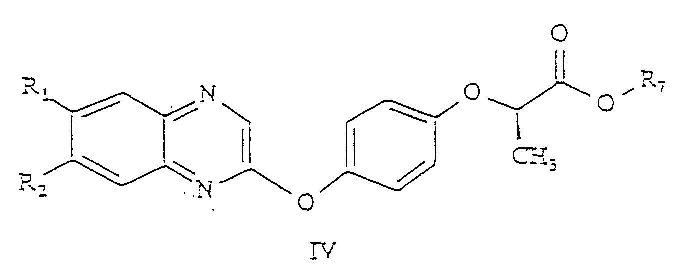 ganz oder überwiegend ohne Racemisierung zur gewünschten Verbindung der Formel I umestert.

2. Verfahren nach Anspruch 1, dadurch gekennzeichnet, dass
R₃ C₁-C₈-Alkyl, Benzyl, C₅-C₆-Cycloalkyl, C₃-C₄-Alkenyl, C₃-C₄-Alkinyl, oder (CH₂)ₙ(O)ₘR₄,
R₄ C₁-C₄-Alkyl, (C₁-C₆-Alkoxy)-C₁-C₄-Alkyl, einen 5- oder 6-gliedrigen gesaettigten oder ungesaettigten Ring, der carbocyclisch oder heterocyclisch mit 0 bis 3 Sauerstoffatomen und 0 bis 2 Stickstoffatomen als Ringatomen von maximal 3 Heteroringatomen ist und unsubstituiert oder durch 1-3 Substituenten aus der Gruppe Oxo, Methyl, Ethyl, n- und i-Propyl, Methoxy, Ethoxy und n- und i-Propoxy substituiert ist, oder N=CR₅R₆,
R₅ und R₆ unabhaengig voneinander C₁-C₄-Alkyl, C₅-C₆-Cycloalkyl, C₁-C₄-Alkylcarbonyloxy-C₁-C₄-alkyl, C₁-C₄-Alkoxy, C₁-C₄-Alkylthio oder zusammen mit dem Kohlenstoffatom, an das sie geknuepft sind, einen Cycloalkylrest mit 5-6 Ringgliedern,
n 1 oder 2 und
m 0 oder 1
bedeuten.

3. Verfahren nach Anspruch 1 oder 2, dadurch gekennzeichnet, daß man als unpolare organische Lösungsmittel bei Raumtemperatur flüssige Kohlenwasserstoffe; die auch halogenhaltig sein können, einsetzt.

4. Verfahren nach einem oder mehreren der Ansprüche 1-3, dadurch gekennzeichnet, dass man als Loesungsmittel Heptan, Toluol, Xylol, Cumol, Mesitylen, Chlorbenzol oder die isomeren Dichlorbenzole verwendet.

5. Verfahren nach einem oder mehreren der Ansprüche 1-4, dadurch gekennzeichnet, dass man die Reaktion von Verbindung II mit Verbindung III in Gegenwart eines Säurebinders durchführt.

6. Verfahren nach Anspruch 5, dadurch gekennzeichnet, dass man als Säurebinder Alkalicarbonate oder Alkalihydrogencarbonate verwendet.

7. Verfahren nach einem der Ansprüche 1 bis 6, dadurch gekennzeichnet, dass man als Katalysatoren langkettige Polyethylenglykole mit einem mittleren Molekulargewicht von 1000 bis 3000 verwendet.

8. Verfahren nach Anspruch 7, dadurch gekennzeichnet, dass man die Katalysatoren in Mengen von 0,1 bis 0,7 mol%, bezogen auf die Verbindung der allgemeinen Formel II, verwendet.

9. Verfahren nach einem oder mehreren der Ansprüche 1-8, dadurch gekennzeichnet,
dass man die Umsetzung von Verbindung der Formel II Verbindung der Formel III bei 30°C bis 150 °C durchfuehrt.

10. Verfahren nach einem oder mehreren der Ansprüche 1-9, dadurch gekennzeichnet, dass man das Zwischenprodukt der genannten Formel IV mit einem 2- bis 100-fachen Ueberschuss des Alkohols der Formel R₃OH, worin R₃ die genannte Bedeutung hat, umestert.

11. Verfahren nach Anspruch 10, dadurch gekennzeichnet, dass man die Umesterung in Gegenwart eines Katalysators aus der Gruppe der Titan-IV-tetraalkoxide durchfuehrt.

12. Verfahren nach Anspruch 11, dadurch gekennzeichnet, dass man den Katalysator in Mengen von 2 bis 50 mol% bezogen auf die Komponente der Formel IV einsetzt.

13. Verfahren nach einem oder mehreren der Ansprüche 10-12, dadurch gekennzeichnet, dass man die Umesterung bei 80 °C bis 120 °C durchfuehrt.

## Claims

1. A process for the preparation of an optically active ester of D-quinoxalinyloxyphenoxypropionic acid of the formula I 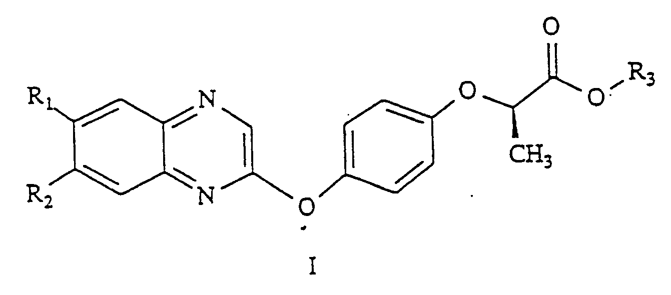 in which
R₁ is H, halogen, C₁-C₄-haloalkyl, CN, NO₂, C₁-C₄-alkyl or C₁-C₄-alkoxy,
R₂ is H or halogen,
R₃ is C₁-C₁₈-alkyl, benzyl, C₅-C₈-cycloalkyl, C₃-C₄-alkenyl which is unsubstituted or substituted by one or more halogen atoms, C₃-C₄-alkynyl which is unsubstituted or substituted by one or more halogen atoms, or (CH₂)ₙ(O)ₘR₄,
R₄ is C₁-C₆-alkyl which is unsubstituted or substituted by C₁-C₆-alkoxy, or is a 5- or 6-membered ring which is saturated or unsaturated, carbocyclic or heterocyclic having 1 to 4 heteroatoms selected from the group consisting of oxygen and nitrogen, and unsubstituted or substituted by 1-3 substituents selected from the group consisting of oxo, C₁-C₃-alkyl and C₁-C₃-alkoxy, or is a group of the formula N=CR₅R₆,
R₅ and R₆ are, independently of each other, C₁-C₆-alkyl, C₃-C₈-cycloalkyl, C₁-C₆-alkylcarbonyloxy-C₁-C₆-alkyl, C₁-C₆-alkoxy, C₁-C₆-alkylthio or, together with the carbon atom to which they are bonded, a cycloalkyl radical having 3-8 ring members,
n is 1 or 2 and
m is 0 or 1,
which comprises
reacting a substituted quinoxaline of the formula II 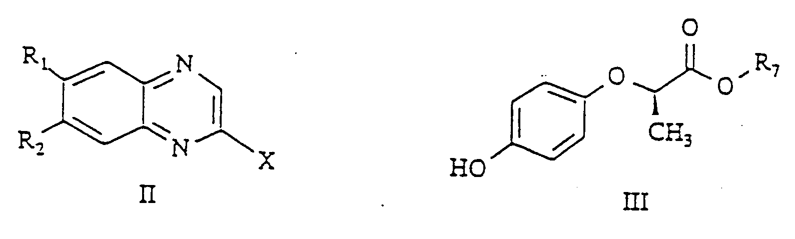 in which
R₁ and R₂ have the same meanings as in formula I and
X is a leaving group, such as e.g. halogen, particularly chlorine or bromine, or methanesulfonyl,
with a (D)-2-(4-hydroxy-phenoxy)propionate of the formula III in which
R₇ is C₁-C₄-alkyl,
in a non-polar organic solvent in the presence of 0.05 to 1.0 mol%, relative to the compound of the formula II, of a catalyst selected from the group consisting of polyethylene glycols and their lower alkyl ethers, and, if R₃ is not identical to R₇, transesterifying the resulting lower alkyl ester of the formula IV or of the formula I, in which R₃=R₇, 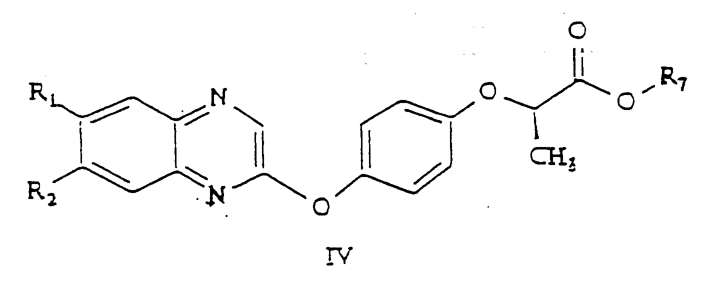 completely or substantially without racemization to give the desired compound of the formula I.

2. The process as claimed in claim 1, wherein
R₃ is C₁-C₈-alkyl, benzyl, C₅-C₆-cycloalkyl, C₃-C₄-alkenyl, C₃-C₄-alkynyl or (CH₂)ₙ(O)ₘR₄,
R₄ is C₁-C₄-alkyl, (C₁-C₆-alkoxy)C₁-C₄-alkyl, or a 5- or 6-membered saturated or unsaturated ring which is carbocyclic or heterocyclic having 0 to 3 oxygen atoms and 0 to 2 nitrogen atoms as ring atoms and at most 3 ring heteroatoms, and unsubstituted or substituted by 1-3 substituents selected from the group consisting of oxo, methyl, ethyl, n- and i-propyl, methoxy, ethoxy, n- and i-propoxy, or is N=CR₅R₆,
R₅ and R₆ are, independently of each other, C₁-C₄-alkyl, C₅-C₆-cycloalkyl, C₁-C₄-alkyl-carbonyloxy-C₁-C₄-alkyl, C₁-C₄-alkoxy, C₁-C₄-alkylthio or, together with the carbon atom to which they are bonded, a cycloalkyl radical having 5-6 ring members,
n is 1 or 2 and
m is 0 or 1.

3. The process as claimed in claim 1 or 2, wherein the non-polar organic solvent employed is a hydrocarbon which is liquid at room temperature and can also contain halogen.

4. The process as claimed in one or more of claims 1-3, wherein the solvent used is heptane, toluene, xylene, cumene, mesitylene, chlorobenzene or an isomeric dichlorobenzene.

5. The process as claimed in one or more of claims 1-4, wherein the reaction of compound II with compound III is carried out in the presence of an acid-binder.

6. The process as claimed in claim 5, wherein the acid-binder used is an alkali metal carbonate or alkali metal hydrogen carbonate.

7. The process as claimed in one of claims 1 to 6, wherein the catalyst used is a long-chain polyethylene glycol having a mean molecular weight of from 1000 to 3000.

8. The process as claimed in claim 7, wherein the catalyst is used in an amount of 0.1 to 0.7 mol%, relative to the compound of the formula II.

9. The process as claimed in one or more of claims 1-8, wherein the reaction of the compound of the formula II with the compound of the formula III is carried out at 30°C to 150°C.

10. The process as claimed in one or more of claims 1-9, wherein the intermediate of the formula IV mentioned is transesterified using a 2- to 100-fold excess of the alcohol of the formula R₃OH in which R₃ has the meaning mentioned.

11. The process as claimed in claim 10, wherein the transesterification is carried out in the presence of a catalyst selected from the group consisting of the titanium(IV) tetraalkoxides.

12. The process as claimed in claim 11, wherein the catalyst is employed in an amount of 2 to 50 mol% relative to the component of the formula IV.

13. The process as claimed in one or more of claims 10-12, wherein the transesterification is carried out at 80°C to 120°C.

## Revendications

1. Procédé pour la préparation des esters optiquement actifs de l'acide D-quinoxalinyloxyphénoxypropionique de formule I : 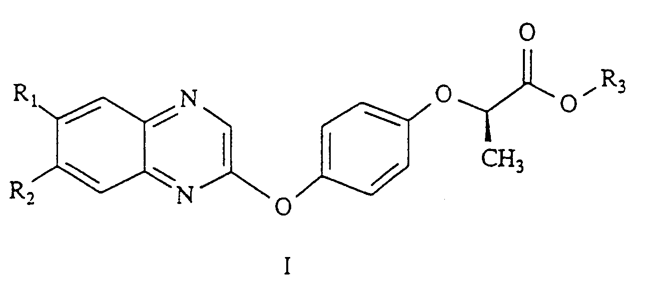 dans laquelle
R₁ représente H, halogène, halogéno-alkyle en C₁-C₄, CN, NO₂, alkyle en C₁-C₄, ou alcoxy en C₁-C₄,
R₂ représente H ou halogène,
R₃ représente alkyle en C₁-C₁₈, benzyle, cycloalkyle en C₅-C₈, alcényle en C₃-C₄, qui est non substitué ou substitué par un ou plusieurs atomes d'halogène, alcynyle en C₃-C₄, qui est non substitué ou substitué par un ou plusieurs atomes d'halogène ou (CH₂)ₙ(O)ₘR₄,
R₄ représente alkyle en C₁-C₆, qui est non substitué ou substitué par alcoxy en C₁-C₆, ou un cycle à 5 ou 6 chaînons, qui est saturé ou insaturé, carbocyclique ou hétérocyclique avec 1 à 4 hétéroatomes pris dans le groupe comportant l'oxygène et l'azote et non substitué ou substitué par 1 à 3 substituants pris dans le groupe comportant oxo, alkyle en C₁-C₃, alcoxy en C₁-C₃, ou représente un groupe de formule générale N=CR₅R₆,
R₅ et R₆, indépendamment l'un de l'autre, représentent alkyle en C₁-C₆, cycloalkyle en C₃-C₈, (alkyl en C₁-C₆)-carbonyloxy-alkyle en C₁-C₆, alcoxy en C₁-C₆, alkylthio en C₁-C₆ ou ensemble avec l'atome de carbone auquel ils sont liés, un radical cycloalkyle avec 3 à 8 chaînons,
n vaut 1 ou 2 et
m vaut 0 ou 1,
caractérisé en ce qu'on fait réagir une quinoxaline substituée de formule générale II : 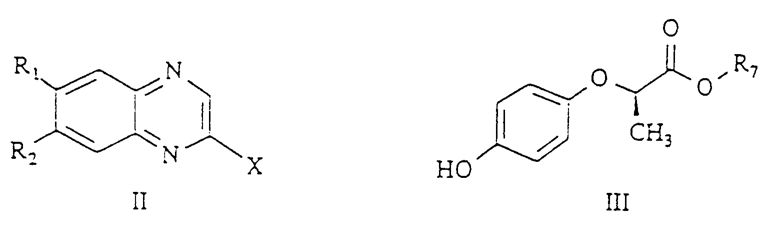 où
R₁ et R₂ ont la même signification que dans la formule I et
X représente un groupe éliminable, comme par exemple halogène, plus particulièrement le chlore et le brome ainsi que le méthanesulfonyle,
avec un (D)-2-(4-hydroxyphénoxy)propionate de formule générale III, où
R₇ représente alkyle en C₁-C₄,
dans un solvant organique non polaire, en présence de 0,05 à 1,0 % en mole par rapport au composé de formule générale II, d'un catalyseur du groupe des polyéthylèneglycols et leurs éthers d'alkyle inférieurs, et dans le cas où R₃ n'est pas égal à R₇, on transestérifie l'ester d'alkyle inférieur ainsi obtenu de formule générale IV, respectivement de formule I où R₃ = R₇ 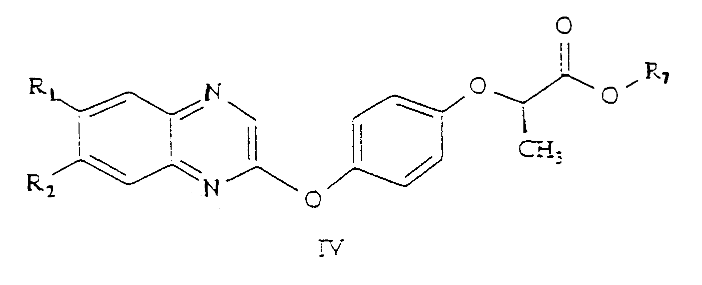 entièrement ou pour la majeure partie sans racémisation pour obtenir le composé voulu de formule I.

2. Procédé selon la revendication 1, caractérisé en ce que
R₃ représente alkyle en C₁-C₈, benzyle, cycloalkyle en C₅-C₆, alcényle en C₃-C₄, alcynyle en C₃-C₄ ou (CH₂)ₙ(O)ₘR₄,
R₄ représente alkyle en C₁-C₄, (alcoxy en C₁-C₆)-alkyle en C₁-C₄, un cycle avec 5 ou 6 chaînons saturé ou insaturé, qui est carbocyclique ou hétérocyclique avec 0 à 3 atomes d'oxygène et 0 à 2 atomes d'azote en tant qu'atomes de cycle avec au maximum 3 hétéroatomes dans le cycle et non substitué ou substitué par 1 à 3 substituants pris dans le groupe des oxo, méthyle, éthyle, n- et i-propyle, méthoxy, éthoxy et n- et i-propoxy, ou représente N=CR₅R₆,
R₅ et R₆, indépendamment l'un de l'autre, représentent alkyle en C₁-C₄, cycloalkyle en C₅-C₆, (alkyle en C₁-C₄)carbonyloxy-alkyle en C₁-C₄, alcoxy en C₁-C₄, alkylthio en C₁-C₄ ou ensemble avec l'atome de carbone auquel ils sont liés ils forment un radical cycloalkyle avec 5 à 6 chaînons,
n vaut 1 ou 2 et
m vaut 0 ou 1.

3. Procédé selon la revendication 1 ou 2, caractérisé en ce qu'on utilise comme solvants organiques non polaires des hydrocarbures liquides à la température ambiante qui peuvent aussi être halogénés.

4. Procédé selon une ou plusieurs des revendications 1 à 3, caractérisé en ce qu'on utilise comme solvants l'heptane, le toluène, le xylène, le cumène, le mésitylène, le chlorobenzène et les dichlorobenzènes isomères.

5. Procédé selon une ou plusieurs des revendications 1 à 4, caractérisé en ce qu'on met en oeuvre la réaction du composé II avec le composé III en présence d'un agent fixant l'acide.

6. Procédé selon la revendication 5, caractérisé en ce qu'on utilise en tant qu'agents fixant l'acide des carbonates alcalins ou des bicarbonates alcalins.

7. Procédé selon une des revendications 1 à 6, caractérisé en ce qu'on utilise en tant que catalyseurs des polyéthylèneglycols à longue chaîne ayant un poids moléculaire moyen de 1000 et 3000.

8. Procédé selon la revendication 7, caractérisé en ce qu'on utilise les catalyseurs à raison de 0,1 à 0,7 % en moles par rapport au composé de formule générale II.

9. Procédé selon une ou plusieurs des revendications 1 à 8, caractérisé en ce qu'on met en oeuvre la réaction du composé de formule II avec le composé de formule III à une température de 30°C à 150°C.

10. Procédé selon une ou plusieurs des revendications 1 à 9, caractérisé en ce qu'on transestérifie le produit intermédiaire de la formule IV citée avec un excès 2 à 100 fois d'alcool de formule R₃OH où R₃ à la signification donnée.

11. Procédé selon la revendication 10, caractérisé en ce qu'on met en oeuvre la transestérification en présence d'un catalyseur pris dans le groupe des tétraalcoxydes de titane-IV.

12. Procédé selon la revendication 11, caractérisé en ce qu'on utilise le catalyseur à raison de 2 à 50 % en moles par rapport au composant de formule IV.

13. Procédé selon une ou plusieurs des revendications 10 à 12, caractérisé en ce qu'on met en oeuvre la transestérification à 80°C à 120°C.
